# EUROPEAN PATENT APPLICATION

(11) **EP 3 533 388 A1**
(43) Date of publication of application: **04.09.2019**
(21) Application number: 18869837.7
(22) Date of filing: 11.06.2018
(51) Int. Cl.: A61B 5/00

(54) **SKIN STATE MEASUREMENT DEVICE USING MULTIPLE LIGHT SOURCES**

(30) Priority: 26.10.2017 KR 20170140489
(71) Applicant: Park, Chang Sik, Anyang-si, Gyeonggi-do 13948 (KR)
(72) Inventor: Park, Chang Sik, Anyang-si, Gyeonggi-do 13948 (KR)
(74) Representative: Beck & Rössig European Patent Attorneys
(86) International application number: PCT/KR2018/006559
(87) International publication number: WO 2019/083116

(57) **Abstract**

A skin state measuring apparatus using a multi-wavelength light source is provided. The apparatus includes a casing configured to allow light of different wavelengths to be selectively irradiated in one direction onto a skin, in contact with the skin at a measurement position by shielding light emitting positions, a reflective capturing unit disposed inside the casing, and configured to capture the skin irradiated with light, while selectively irradiating visible light, ultraviolet (UV) light, and infrared (IR) light as reflection-based indirect light onto the skin in the one direction, a direct light sensing unit disposed inside the casing, at one side of the reflective capturing unit, and configured to sense a state of the skin, while irradiating the skin with direct light emitted from the reflective capturing unit and passed therethrough, a control unit disposed inside the casing, and configured to control capturing and transmission of an image by controlling the reflective capturing unit and the direct light emitting unit to irradiate light selected from among the visible light, the UV light, and the IR light in the form of indirect light onto the skin and to irradiating penetrate direct light onto the skin according to the state of the skin, and a capture analysis unit disposed at one side of the casing, coupled electrically to the control unit, and configured to synthesize images captured by the control unit according to the state of the skin and analyze the synthesized image by color and shape comparison.

## Description

### [Technical Field]

The present disclosure relates to a skin state measuring apparatus using a multi-wavelength light source, and more particularly, to a skin state measuring apparatus using a multi-wavelength light source, which allows visual check during measurement to pre-check a state of the skin and the symptoms of skin disorders by analyzing an image of information such as the shape, size, and color of a pigmented lesion inside the skin, which has been captured by irradiating multi-wavelength light including visible light, ultraviolet (UV) light, and infrared IR) light, thereby increasing measurement accuracy and providing basic data for skin diagnosis according to the depth and shape of the pigmented lesion.

### [Background Art]

In general, the skin, which is an organ that protects muscles and other organs in the human body, plays a very critical role in protecting the human body from germs. Further, other important functions of the skin include insulation, body temperature control, a sensory function, vitamin D synthesis, and the protection function of vitamin B and folates.

The skin protects the human body from germs, in direct contact with ambient air. As the skin is an outer organ of the human body and thus is brought into direct contact with external impurities, the impurities may be accumulated in hair roots or produce sebum or blackheads. When the accumulated impurities are introduced into the skin, the skin is pigmented and thus damaged. Therefore, the skin is treated or cured by measuring the shape and depth of the impurity, using a skin measuring device.

Further, if sunlight is irradiated onto the skin, the above-described important functions are executed, which are favorable for health. However, long-time exposure to sunlight may cause pigmentation, sun burns, dermatoheliosis, and so on. In an extreme case, a skin cancer may be caused by melanoma in the form of a spot.

Accordingly, there is a pressing need for diagnosing skin diseases such as nevus, tumors, leukoplakia, melanoma, and wound as well as measuring a skin state for esthetic purposes, such as hair roots, pigmentation, sebum, and blackheads.

Conventionally, a skin measuring device that irradiates multi-wavelength light by means of a filter was developed and has been used. In the conventional skin measuring device, if UV light is irradiated onto the skin and a camera sensor unit equipped with a UV filter makes only visible rays seen through a camera, porphyrin and melanin excited by UV light and irradiated by visible light can be observed.

However, the joint use of a polarization filter and a UV filter leads to over-blocking of light, thereby making the camera too dark and thus making it difficult to maximize the UV light irradiation effect.

Further, when the skin is observed while changing cameras equipped with different light sources, it is not easy to observe the same site on the skin. Additionally, long-time operation rapidly shortens the lifetime of a white light source and a UV light source. Particularly, UV light may cause melanin pigmentation and skin damage. Accordingly, the conventional skin observation device has limitations in observing one specific site of the skin with various light sources. Moreover, long-time operation rapidly shortens the lifetime of a used light source and long-time UV irradiation onto the skin may cause melanin pigmentation and skin damage.

### [Disclosure]

### [Technical Problem]

An aspect of the present disclosure devised to solve the problem lies on a skin state measuring apparatus using a multi-wavelength light source, which allows visual check during measurement to pre-check a state of the skin and the symptoms of skin disorders by analyzing an image of information such as the shape, size, and color of a pigmented lesion inside the skin, which has been captured by irradiating multi-wavelength light including visible light, ultraviolet (UV) light, and infrared IR) light, thereby increasing measurement accuracy and providing basic data for skin diagnosis according to the depth and shape of the pigmented lesion.

It will be appreciated by persons skilled in the art that the objects that could be achieved with the present disclosure are not limited to what has been particularly described hereinabove and the above and other objects that the present disclosure could achieve will be more clearly understood from the following detailed description.

### [Technical Solution]

In an aspect of the present disclosure, a skin state measuring apparatus using a multi-wavelength light source includes a casing configured to allow light of different wavelengths to be selectively irradiated in one direction onto a skin, in contact with the skin at a measurement position by shielding light emitting positions, a reflective capturing unit disposed inside the casing, and configured to capture the skin irradiated with light, while selectively irradiating visible light, ultraviolet (UV) light, and infrared (IR) light as reflection-based indirect light onto the skin in the one direction, a direct light sensing unit disposed inside the casing, at one side of the reflective capturing unit, and configured to sense a state of the skin, while irradiating the skin with direct light emitted from the reflective capturing unit and passed therethrough, a control unit disposed inside the casing, and configured to control capturing and transmission of an image by controlling the reflective capturing unit and the direct light emitting unit to irradiate light selected from among the visible light, the UV light, and the IR light in the form of indirect light onto the skin and to irradiating penetrate direct light onto the skin according to the state of the skin, and a capture analysis unit disposed at one side of the casing, coupled electrically to the control unit, and configured to synthesize images captured by the control unit according to the state of the skin and analyze the synthesized image by color and shape comparison.

The casing unit may include a casing body configured to contact the skin during skiing measurement, thereby allowing light irradiation by a user's manipulation and including, therein, a casing space opened at one side and accommodating the reflective capturing unit, the direct light sensing unit, and the control unit, and a shielding body disposed at one side of the casing body, and including a shielding space formed at a position other than a shielding opening partially opened at a center of one side of the shielding space, with the reflective capturing unit installed in the other direction inside the shielding body, for shielding light irradiated from the reflective capturing unit, direct light emission holes at a plurality of positions at a portion of the shielding space, for allowing parts irradiating IR light of different wavelengths of the direct light sensing unit to be inserted therethrough, and a sensor installation hole for allowing a skin sensing part of the direct light sensing unit to be inserted therethroguh.

The reflective capturing unit may include a light blocking body disposed inside the casing and configured to close an opened other part of a light blocking part of the casing and emit light in the other direction, reflective light emitters arranged, apart from one side of the light blocking body by a predetermined distance, at a plurality of positions of an outer periphery of the light emitting body apart from a center the light emitting body by a predetermined distance, to selectively irradiate the visible light, the UV light, and the IR light in the other direction, coupled to the control unit to be controlled by the control unit, and configured to selectively irradiate the visible light, the UV light, and the IR light in the other direction according to a control signal, a reflection body disposed between the light blocking body and the reflective light emitters, and including a reflection space for accommodating the reflective light emitters therein, the reflection space being curved, surrounding the other side of the reflective light emitters and being opened at one side thereof, a reflection surface inside the reflection space, for reflecting light irradiated in the other direction by the reflective light emitters in the one direction, thereby refracting the light in the one direction, and a reflection through hole penetrating through the center of the reflection body, and a capturing module fixed disposed at one side of the light blocking body, inserted into the reflection through hole, and configured to capture the skin in a state where light irradiated from the reflective light emitters and reflected from the reflection body and light directly emitted from the direct light sensing unit are selectively irradiated onto the skin.

The reflective capturing unit may include a light blocking body disposed inside the casing and configured to close an opened other part of a light blocking part of the casing and emit light in the other direction, reflective light emitters arranged, apart from one side of the light blocking body by a predetermined distance, at a plurality of positions of an outer periphery of the light emitting body apart from a center the light emitting body by a predetermined distance, to selectively irradiate the visible light, the UV light, and the IR light in the other direction, coupled to the control unit to be controlled by the control unit, and configured to selectively irradiate the visible light, the UV light, and the IR light in the other direction according to a control signal, a reflection body disposed between the light blocking body and the reflective light emitters, and including a reflection space for accommodating the reflective light emitters therein, the reflection space being curved, surrounding the other side of the reflective light emitters and being opened at one side thereof, a reflection surface inside the reflection space, for reflecting light irradiated in the other direction by the reflective light emitters in the one direction, thereby refracting the light in the one direction, and a reflection through hole penetrating through the center of the reflection body, a capturing mirror disposed between the light blocking body and the reflection body, at one portion apart from the reflection through hole by a predetermined distance, and configured to refract a captured image from a center in one side direction, and a capturing module disposed inside the casing, provided to capture an image refracted from the capturing mirror, at a position for capturing from one side surface toward the center, and configured to capture the skin in a state where light irradiated from the reflective light emitters and reflected from the reflection body and light directly emitted from the direct light sensing unit are selectively irradiated onto the skin.

The reflective capturing unit may include a light blocking body disposed inside the casing and configured to close an opened other part of a light blocking part of the casing and emit light in the other direction, reflective light emitters arranged, apart from the one side of the light blocking body by a predetermined distance, at a plurality of positions of an outer periphery of the light emitting body apart from a center the light emitting body by a predetermined distance, to selectively irradiate the visible light, the UV light, and the IR light in the other direction, coupled to the control unit to be controlled by the control unit, and configured to selectively irradiate the visible light, the UV light, and the IR light in the other direction according to a control signal, diffusion plates disposed between the reflective light emitters and the skin, at one side of the reflective light emitters, and configured to diffuse the light emitted from the reflective light emitters in an expanded light irradiation range and irradiating the light in the form of indirect light onto the skin, and a capturing module fixed at the center of a portion of the light blocking body, apart from the other side of the reflective light emitters by a predetermined distance, and configured to capture the skin in a state where light diffused by the diffusion plates and light directly emitted from the direct light sensing unit are selectively irradiated onto the skin.

The direct light sensing unit may include a direct light blocking body disposed at the one side of the reflective capturing unit, positioned at one shielded side of the casing, on which the reflective capturing unit is installed, and including a direct light blocking through hole penetrating through the center thereof, for exposing reflected light of the reflective capturing unit and the captured skin therethrough, a plurality of direct light emitters arranged at one side of the direct light blocking body, provided around an outer periphery of the direct light blocking through hole to emit IR light, inserted to protrude outward from a part of the casing shielding the reflective capturing unit, and configured to irradiate light directly onto the skin, and a sensor disposed at the one side of the direct light blocking body, inserted to protrude outward from the part of the casing shielding the reflective capturing unit, and configured to measure the state of the skin in the vicinity of the skin.

The direct light sensing unit may include a direct light blocking body disposed at the one side of the reflective capturing unit, positioned at one shielded side of the casing, on which the reflective capturing unit is installed, and including a direct light blocking through hole penetrating through the center thereof, for exposing reflected light of the reflective capturing unit and the captured skin therethrough, a plurality of direct light emitters arranged at one side of the direct light blocking body, provided around an outer periphery of the direct light blocking through hole to emit IR light in a plurality of different wavelengths, inserted to protrude outward from a part of the casing shielding the reflective capturing unit, and configured to irradiate light directly onto the skin, and a sensor disposed at the one side of the direct light blocking body, inserted to protrude outward from the part of the casing shielding the reflective capturing unit, and configured to measure the state of the skin in the vicinity of the skin.

The direct light sensing unit may include a direct light blocking body disposed at the one side of the reflective capturing unit, positioned at one shielded side of the casing, on which the reflective capturing unit is installed, and including a direct light blocking through hole penetrating through the center thereof, for exposing reflected light of the reflective capturing unit and the captured skin therethrough, a plurality of direct light emitters arranged at one side of the direct light blocking body, provided around an outer periphery of the direct light blocking through hole to emit IR light in a plurality of different wavelengths and visible light, inserted to protrude outward from a part of the casing shielding the reflective capturing unit, and configured to irradiate light directly onto the skin, and a sensor disposed at the one side of the direct light blocking body, inserted to protrude outward from the part of the casing shielding the reflective capturing unit, and configured to measure the state of the skin in the vicinity of the skin.

The sensor may include at least one of an oil sensor, a moisture sensor, an elasticity sensor a temperature sensor, an ultrasonic sensor, and a PH sensor.

The control unit may include a control switch protruding from one side surface of the casing and configured to, upon user pressing to control light output and capturing, generate a control signal, a controller disposed inside the casing, coupled to the control switch, and configured to determine whether to selectively irradiate the visible light, the UV light, and the IR light emitted from the reflective capturing unit according to a manipulation signal and generate an indirect light emission control signal, to determine whether to directly irradiate light penetrating into the skin from the direct light sensing unit and generate a direct light emission control signal, to determine whether to capture an image according to a combination of light emitted from the reflective capturing unit and light emitted from the direct sensing unit and generate a capture signal indicating capturing, and to generate a measurement control signal to selectively measure a part of the skin, sensed by the direct light sensing unit, a reflected visible light control unit disposed inside the casing, and configured to control the reflective capturing unit to emit reflected visible light in the form of indirect light according to the indirect light emission control signal generated from the controller coupled to the reflective capturing unit, a reflected UV light control unit disposed inside the casing, and configured to control the reflective capturing unit to emit reflected UV light in the form of indirect light according to the indirect light emission control signal generated from the controller coupled to the reflective capturing unit, a reflected IR light control unit disposed inside the casing, and configured to control the reflective capturing unit to emit reflected IR light in the form of indirect light according to the indirect light emission control signal generated from the controller coupled to the reflective capturing unit, a direct IR light control unit disposed inside the casing, and configured to control the direct light sensing unit to emit direct IR light of a selected wavelength from among the plurality of different wavelengths according to the direct light emission control signal generated from the controller coupled to the direct light sensing unit, a capturing control unit disposed inside the casing and configured to control capturing of the skin according to the capture signal generated from the controller coupled to the refractive capturing unit and transmit the captured image to the capture analysis unit, and a sensor control unit disposed inside the casing and configured to control measurement of the state of the skin by a measurement sensor selected in the direct light sensing unit according to the measurement control signal generated from the controller coupled to the reflective capturing unit.

The capture analysis unit may include a screen synthesis unit disposed at the one side of the casing, and configured to receive a plurality of images from the control unit coupled to a capturing part of the reflective capturing unit and synthesize the received plurality of images under a condition that indirect light irradiated from the reflective capturing unit and direct light irradiated from the direct light sensing unit are controlled by the control unit and irradiated as multi-wavelength light, a color comparison unit disposed at the one side of the casing, coupled to the screen synthesis unit, and configured to diagnose a material generated in the skin by comparing the synthesized image with basic color information generated for respective colors according to the synthesized image and a type of the material generated in the skin, and a shape comparison unit disposed at the one side of the casing, coupled to the screen synthesis unit, and configured to diagnose the material generated in the skin by comparing the synthesized image with basic shape information generated for respective shapes according to the synthesized image and the type of the material generated in the skin.

In another aspect of the present disclosure, a skin state measuring apparatus using a multi-wavelength light source includes a light emitter disposed with distinguishable light sources of different wavelengths to be selectively irradiated to a measurement position of a skin, a capturing module configured to capture the measurement position, while light is irradiated from a light source, a casing body configured to shield direct irradiation of light from the light sources of the different wavelengths to the capturing module, a control unit disposed inside the casing body and configured to control irradiation of the light emitter and an operation of the capturing module, and a capture analysis unit configured to synthesize a plurality of images captured at the surface of the skin to a predetermined depth into the skin at the measurement position and generate the synthesized image along a depth axis. The capture analysis unit obtains information about the presence or absence of a spot at the measurement position and the size, shape, and color of the spot by analyzing the synthesized image, and determines the depth of the spot or whether the spot is a melanin disorder forming a nevus.

### [Advantageous Effects]

The skin state measuring apparatus using a multi-wavelength light source according to the embodiments of the present disclosure allows visual check during measurement to pre-check a state of the skin and the symptoms of skin disorders by analyzing an image of information such as the shape, size, and color of a pigmented lesion inside the skin, which has been captured by irradiating multi-wavelength light including visible light, ultraviolet (UV) light, and infrared IR) light, thereby increasing measurement accuracy and providing basic data for skin diagnosis according to the depth and shape of the pigmented lesion.

Further, the skin state measuring apparatus using a multi-wavelength light source according to the embodiments of the present disclosure selectively irradiates multi-wavelength light of visible light, UV light, and IR light in the form of indirect light through reflection, and selectively irradiates IR light of different wavelengths, which penetrates deep into the skin, in the form of direct light in the vicinity of the skin, such that images of an object under measurement on the surface of the skin and deep into the skin are captured by differentiating wavelengths according to depths in the skin. Accordingly, the size, color, and shape of the objects under measurement can be measured from the skin surface to depths into the skin.

Further, the skin state measuring apparatus using a multi-wavelength light source according to the embodiments of the present disclosure captures an object under measurement on the skin surface and at a certain depth by means of a multi-wavelength light source for visible light, UV light, and IR light, and captures images according to depths into the skin by selectively irradiating a plurality of IR rays in different wavelengths directly onto the skin. Therefore, as the apparatus captures images while changing light without using a filter, the apparatus is simplified in structure and increases use convenience.

In addition, the skin state measuring apparatus using a multi-wavelength light source according to the embodiments of the present disclosure renders the color, size, and shape of an object under measurement as an image by synthesizing images captured on the skin surface and at different depths into the skin through multi-wavelength light. Therefore, the skin can be diagnosed by comparing the state of the skin with samples related to diseases, thereby increasing use convenience.

### [Description of Drawings]

The accompanying drawings, which are included to provide a further understanding of the disclosure, illustrate embodiments of the invention and together with the description serve to explain the principle of the disclosure.

In the drawings:
FIG. 1 is a perspective view illustrating a skin state measuring apparatus using a multi-wavelength light source according to an embodiment of the present disclosure;
FIG. 2 is an exploded perspective view illustrating the skin state measuring apparatus using a multi-wavelength light source illustrated in FIG. 1, in which some part is not shown;
FIG. 3 is a block diagram illustrating an operational relationship between main functions in the skin state measuring apparatus using a multi-wavelength light source illustrated in FIG. 1;
FIG. 4 is a diagram illustrating operational states of main components in the skin state measuring apparatus using a multi-wavelength light source illustrated in FIG. 1;
FIG. 5 is a diagram illustrating operational states of main components in a skin state measuring apparatus using a multi-wavelength light source according to another embodiment of the present disclosure;
FIG. 6 is a diagram illustrating operational states of main components in a skin state measuring apparatus using a multi-wavelength light source according to another embodiment of the present disclosure;
FIG. 7 illustrates pictures of images of different skin symptoms, taken by means of a multi-wavelength light source in a skin state measuring apparatus using a multi-wavelength light source according to an embodiment of the present disclosure;
FIG. 8 illustrates pictures of a plurality of images captured for skin analysis in a skin state measuring apparatus using a multi-wavelength light source according to an embodiment of the present disclosure;
FIG. 9 illustrates analysis state pictures for an image obtained by synthesizing the images of FIG. 8 in an image analysis method for skin analysis;
FIG. 10 illustrates analysis state pictures in an example of synthesizing and analyzing the images of FIG. 8 according to symptoms;
FIG. 11 illustrates pictures of images of spots generated on the skin, captured by a skin state measuring apparatus using a multi-wavelength light source according to an embodiment of the present disclosure; and
FIG. 12 is a diagram illustrating a process of rendering the size and shape of a spot as a three-dimensional (3D) image by arranging the captured images of FIG. 11 according to depths into the skin and synthesizing the images.

### <Description of Reference Numerals for Important Components in the Drawings>

- 100:: measuring apparatus
- 110:: casing
- 111:: casing body
- 112:: casing space
- 113:: shielding body
- 114:: shielding space
- 115:: shielding opening
- 116:: direct light emission hole
- 117:: sensor installation hole
- 120:: reflective capturing unit
- 121:: light blocking body
- 122:: reflective light emitter
- 123:: reflection body
- 124:: reflection space
- 125:: reflection surface
- 126:: reflection through hole
- 127:: camera module
- 128:: capturing mirror
- 129:: diffusion plate
- 130:: direct light sensing unit
- 131:: direct light blocking body
- 132:: direct light blocking through hole
- 133:: direct light emitter
- 134:: sensor
- 140:: control unit
- 141:: control switch
- 142:: controller
- 143:: reflected visible light control unit
- 144:: reflected UV light control unit
- 145:: reflected IR light control unit
- 146:: direct IR light control unit
- 147:: capturing control unit
- 148:: sensor control unit
- 150:: capture analysis unit
- 151:: screen synthesis unit
- 152:: color comparison unit
- 153:: shape comparison unit

### [Best Mode]

Various embodiments of the present disclosure are described with reference to the accompanying drawings. However, the scope of the present disclosure is not intended to be limited to the particular embodiments, and it is to be understood that the present disclosure covers various modifications, equivalents, and/or alternatives.

Lest it should obscure the subject of the present disclosure, a detailed description of known techniques will be avoided herein. Further, the term as used in the present disclosure, "1^{st}", "2^{nd}", "first" or "second' are used to distinguish one component from another component.

When it is said that a component is "(operatively or communicatively) coupled with/to" or "connected to" another component, it should be understood that the one component is connected to the other component directly or through any other component.

The terms "module" and "unit" used to signify components are used herein to help the understanding of the components and thus they should not be considered as having specific meanings or roles. Accordingly, the terms "module" and "unit" may be used interchangeably. In addition, a part that is not relevant to the description is not shown in the drawings in order to make the present disclosure clear, and the widths, lengths, and thickness of components are much simplified and may not be drawn to scale, and their sole purpose is to facilitate easy and clear explanation of the embodiments. Like reference numerals denote the same components throughout the specification.

Reference will now be made in detail to the preferred embodiments of the present invention, examples of which are illustrated in the accompanying drawings.

With reference to the attached drawings, embodiments of the present disclosure will be described below in detail.

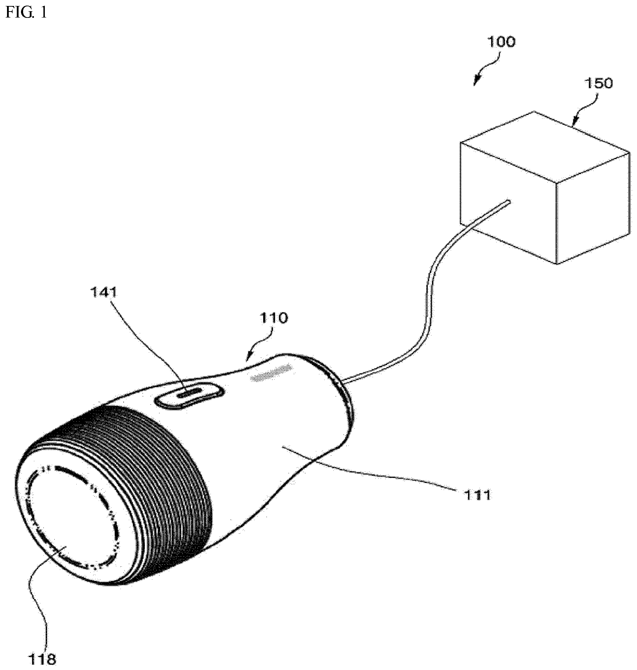

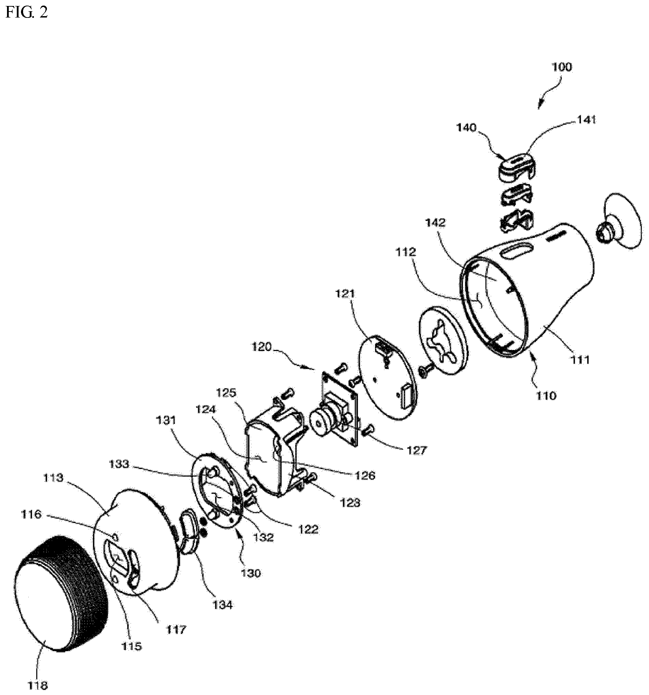

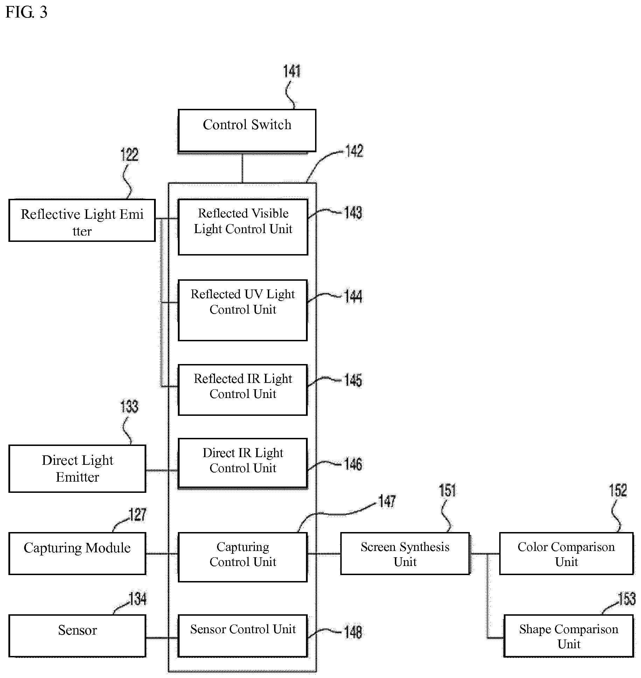

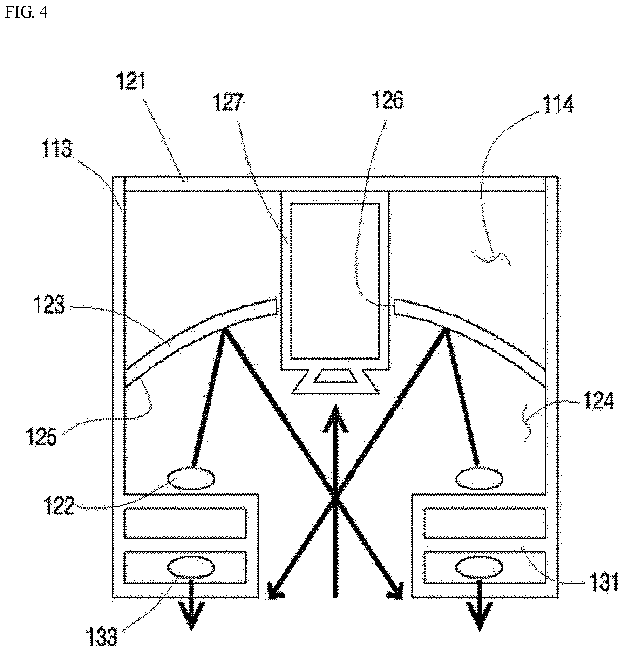

FIG. 1 is a perspective view illustrating a skin state measuring apparatus using a multi-wavelength light source according to an embodiment of the present disclosure, FIG. 2 is an exploded perspective view illustrating the skin state measuring apparatus using a multi-wavelength light source illustrated in FIG. 1, in which some part is not shown, FIG. 3 is a block diagram illustrating an operational relationship between main functions in the skin state measuring apparatus using a multi-wavelength light source illustrated in FIG. 1, and FIG. 4 is a diagram illustrating operational states of main components in the skin state measuring apparatus using a multi-wavelength light source illustrated in FIG. 1.

Referring to FIG. 1 to 4, a skin state measuring apparatus 100 using a multi-wavelength light source according to an embodiment of the present disclosure is designed to measure a skin state and the shape of a lesion caused by a skin disorder and thus to eliminate an impurity from the skin, such as hair roots, pigmentation, sebum, and blackheads and diagnose a skin disease such as a nevus, tumors, leukoplakia, melanoma, and wound.

Particularly, melanoma is a kind of tumor. If the melanoma is benign, it has only to be removed for esthetic purposes because it is not harmful. However, if melanoma is malignant, the melanoma is diagnosed as cancer, thereby threatening life in some cases.

Risk of malignancy of melanoma is assessed by the ABCDE rule of dermoscopy ([A]=asymmetry, [B]=border irregularity, [C]=color variegation, [D]=diameter, [E]=evolution).

Asymmetry (Is the shape asymmetrical?): melanoma is asymmetrical. If a skin lesion is asymmetrical, the skin lesion may be highly suggestive of melanoma.

Border irregularity (Is the border irregular?): a spot (melanocytic nevus) has a smoothly curved outline like a circle, while melanoma has a rugged and irregular outline.

Color variegation (Does it have various colors?): if one lesion has two or more colors and shades such as brown and black, the lesion is highly suggestive of melanoma.

Diameter (Is the diameter equal to or larger than 0.6cm?): most spots are not larger than 0.6cm in size. If the size of a recent spot exceeds 0.6cm, the spot is likely to be melanoma.

Elevation or evolution (Does a mole become raised or have a changed color or size?): if a spot becomes thick and raised or its color or size suddenly changes or increases, the spot is highly likely to be melanoma.

Further, a kind of skin pigment, melanin protects the skin against UV light. With too much melanin secretion, the color of hair roots becomes dark or symptoms such as pigmentation or sebum occur. Thus, as the skin state gets poor, the area and depth of such a lesion may be measured, for use as data for skin care or treatment.

If melanin is measured by irradiating IR light which penetrates deep into the skin, it is difficult to determine accurate information about the shape and color of a lesion. Thus, an image obtained by irradiating visible light is used.

However, an image captured by visible light irradiation is only about the surface of the skin because visible light does not penetrate deep into the skin. Therefore, although the color and shape of a lesion may be accurately determined, it is difficult to determine how deep the lesion is in the skin.

Therefore, since the color, shape, and depth of the melanin pigment are accurately detected by synthesizing an image captured by IR light that penetrates into the skin and an image captured by visible light irradiation, a skin state measuring apparatus using a multi-wavelength light source is used.

With regard to melanoma which is deep in the form of a spot inside the skin as illustrated in [Table 1] and thus is not identified by the shape and color of a skin surface , the skin state measuring apparatus 100 using a multi-wavelength light source according to the embodiment of the present disclosure captures the shape and color of the skin surface by visible light and UV light, captures an image of the skin at each depth by IR light that penetrates deep into the skin, synthesizes the images into one image, and compares the synthesized image in shape and color.

Further, since it is difficult to identify skin damage caused by melanin, such as pigmentation by IR light, the skin state measuring apparatus 100 using a multi-wavelength light source captures a skin surface by visible light and synthesizes images captured at different depths by IR light.

This skin state measuring apparatus 100 using a multi-wavelength light source includes a casing 110, a reflective capturing unit 120, a direct light sensing unit 130, a control unit 140, and a capture analysis unit 150.

The casing 110 is provided to shield each light emission position such that light of different wavelengths may be selectively irradiated onto the skin in one direction, in contact with a measurement position of the skin.

The casing 110 includes a casing body 111 with various parts built therein, which contacts the skin, and a shielding body 113.

The casing body 111 irradiates light by a user's manipulation, in contact with the skin during skin measurement. The casing body 111 includes a casing space 112 open at one side thereof, in which the reflective capturing unit 120, the direct light sensing unit 130, and the control unit 140 are accommodated.

That is, the reflective capturing unit 120, the direct light sensing unit 130, and the control unit 140 are installed in the casing body 111. When the user contacts the casing body 111 on the skin, while grabbing the casing body 111 and manipulates the control unit 140, the reflective capturing unit 120, the direct radiation sensing unit 130 are operated.

The shielding body 113 is disposed at one side of the casing body 111, and includes a shielding space 114 formed at a position other than a shielding opening 115 partially opened at a center of one side of the shielding space 114, with the reflective capturing unit 120 installed in the other direction inside the shielding body 114, for shielding light irradiated from the reflective capturing unit 112.

The shielding body 113 is formed in the form of a space in which the partially opened shielding opening 115 is installed, at one side thereof, and is shielded at the other opened side thereof by the reflective capturing unit 120. To capture an image under the control of the controller 140, the shielding body 113 blocks light of the other parts so as to allow light irradiation toward the shielding opening 115.

At a portion of the shielding body 113, direct light emission holes 116 are formed at a plurality of positions, for allowing parts irradiating IR light of different wavelengths of the direct light sensing unit 130 to be inserted therethrough, and a sensor installation hole 117 is formed for allowing a skin sensing part of the direct light sensing unit 113 to be inserted therethroguh. To allow insertion of and support the direct light sensing unit 130 provided at one side of the shielding body 113, the direct light emission hole 116 is formed at a position in which direct light is irradiated, and the sensor installation hole 117 is formed at a position in which biometric information is sensed, on one side surface of the shielding body 113.

The reflective capturing unit 120 is disposed inside the casing 110, and captures an image of a skin onto which light is irradiated, while selectively irradiating visible light, UV light, and IR light in the form of indirect light in one direction.

The reflective capturing unit 120 includes a light blocking body 121 disposed at the other side of the shielding body 113, for blocking light, reflective light emitters 122, a reflection body 123, and a capturing module 127.

The light blocking body 121 is disposed at the other side of the shielding body 113, and installed to close the opened other side of the shielding body 113. Thus, the light blocking body 121 blocks the opened other side in order to irradiate light to the outside through the shielding opening 115, while blocking the light in the shielding space 114.

The reflective light emitters 122 are disposed apart from one side of the light blocking body 121 by a predetermined distance. In a state where the reflective light emitters 122 are provided at a plurality of positions around an outer periphery apart from a center by a predetermined distance, to irradiate visible light, UV light, and IR light toward the other side, the reflective light emitters 122 are coupled to the control unit 140, and selectively irradiate visible light, UV light, and IR light in the other direction. A plurality of reflective light emitters 122 are arranged around the outer periphery of the shielding opening 115 from one direction inside the shielding body 113 in order to irradiate light toward the reflection body 123.

To irradiate optimum light according to the type, shape, and color of an object under measurement in the skin, the reflective light emitters 122 select one of visible light, UV light, and IR light, for capturing an image. When different light is needed, the reflective light emitters 122 select one of the above lights and capture an image. As such, an environment in which an image is captured by means of a multi-wavelength light source is built, an image of the object under measurement in its accurate color, size, and shape may be obtained.

That is, visible light is light in a spectrum visible to the human eye. With the visible light, the color and size of an object under measurement on a surface may be found. With irradiation of UV light, pigmentation such as melanin pigmentation may be found. IR light penetrates into the skin, and thus depths in the skin are known according to wavelengths. As such, the use of the multi-wavelength light source enables capturing of an image of the object under measurement in an accurate measurement of the color and size of the object under measurement.

The reflection body 123 is disposed between the light blocking body 121 and the reflective light emitters 122. The reflection body 123 is curved to surround the other side of the reflective light emitters 122. The reflection body 123 is opened at one side thereof, having a reflection space 124 in which the reflective light emitters 122 are positioned. A reflection surface 125 is formed inside the reflection space 124, to reflect light irradiated in the other direction by the reflective light emitters 122 so that the light may be refracted in the one direction. A reflection through hole 126 is formed at the center of the reflection surface 125.

That is, the reflection body 123 is provided to reflect light irradiated in the other direction by the reflective light emitters 122 so that the light may be refracted in the one direction. Thus, the reflection body 123 enables irradiation of indirect light onto the skin through reflection, instead of direct irradiation, thereby preventing production of light noise caused by light scattering or blur.

The skin is an organ containing oil and moisture. When the skin is captured by irradiating light directly, the flare phenomenon that light is reflected and scattered inside a camera occurs. Therefore, the resulting ghost phenomenon of double image formation during capturing or the fog phenomenon of generation of misty noise like fog makes it difficult to capture an accurate image.

As light emitted from the reflective light emitters 122 is primarily reflected from the reflection body 123 and the resulting indirect light is irradiated onto the skin, light available for capturing is provided, while the ghost phenomenon and the fog phenomenon are minimized.

Further, since the reflection body 123 is curved and reflects light irradiated from the reflective light emitters 122, light may be spread over a wide range through reflection even with the use of a low-power light source. Hence, even though the output of the reflective light emitters 122 is lowered, light may be used through diffusion.

The capturing module 127 is fixedly arranged at one side of the light blocking body 121, and inserted into the reflection through hole 126, to capture an image of the skin irradiated with light emitted from the reflective light emitters 122 and reflected from the reflection body 123.

The capturing module 127 captures the skin by selectively irradiating indirect light reflected from the reflection body 123 and direct light emitted from the direct light sensing unit 130, to thereby obtain an image of an object under measurement in its size, shape, and color.

The direct light sensing unit 130 is disposed inside the casing 110, at one side of the reflective capturing unit 120. As light irradiated from the reflective capturing unit 120 passes through the direct light sensing unit 130, IR light of different wavelengths at different positions is irradiated onto the skin.

The direct light sensing unit 130 includes a direct light blocking body 131 for blocking irradiation of light to the other direction inside the shielding body 113, direct light emitters 133, and a sensor 134.

The direct light blocking body 131 is disposed at one side of the reflective light emitters 122, and provided with a direct light blocking through hole 132 positioned at one end inside the shielding body 113 and communicating with the shielding opening 115 at the center. The direct light blocking body 131 is supportedly installed to supply power to the direct light emitters 133. The direct light blocking body 131 blocks introduction of light emitted from the direct light emitters 133 toward the reflective light emitters 122 in the other direction.

A plurality of direct light emitters 133 are arranged on one side of the direct light blocking body 131. The plurality of direct light emitters 133 are provided around an outer periphery of the direct light blocking through hole 132 to emit IR light in different wavelengths. The direct light emitters 133 are inserted into a direct light emission hole penetrating through one side of the reflection body 123, protruding in the one direction, so as to directly irradiate light onto the skin in the one direction, while being blocked at the other side thereof.

The direct light emitters 133 are installed to directly irradiate light into the skin so that the capturing module 127 may capture an object at a configured depth irradiated by IR light.

The direct light emitters 133 may be embodied in various embodiments according to a state of the skin and the type of an object under measurement. In an embodiment, a single direct light emitter 133 is provided to irradiate IR light of a single wavelength. Further, in another embodiment, a plurality of direct light emitters 133 are provided to irradiate IR light in different wavelengths at different positions. Under the control of the control unit 140, the direct light emitters 133 may irradiate IR light of a selected wavelength. In a third embodiment, a plurality of direct light emitters 133 are provided to irradiate IR light and visible light in different wavelengths. Under the control of the control unit 140, the direct light emitters 133 may irradiate IR light or visible light of a selected wavelength.

The embodiments of the direct light emitters 133 differ from each other only in terms of the types or number of light sources, and identical to each other in terms of operational relationships. Accordingly, only the direct light emitters 133 for emitting IR light in different wavelengths according to the foregoing second embodiment will be described. This is intended only for the convenience of description, and it is apparent to those skilled in the art that the following description is also applied to the direct light emitters 133 according to the first and third embodiments.

The direct light emitters 133 according to the second embodiment irradiate IR light which is capable of penetrating into the skin, compared to other types of light. As the depth of the IR light is controllable according to its wavelength, the plurality of direct light emitters 133 are coupled to the control unit 140 to irradiate IR light in different wavelengths. A direct light emitter 133 of a wavelength corresponding to a user-desired depth is selected by a control signal from the control unit 40, and irradiates IR light so that an object under measurement may be captured according to its depth.

The sensor 134 is disposed at one side of the direct light blocking body 131 and inserted into the sensor installation hole 117 formed in the form of a through hole on one side surface of the shielding body 113, protruding in the one direction, to capture the state of the skin in the vicinity of the skin.

The sensor 134 includes at least one of an oil sensor, a moisture sensor, a temperature sensor, an ultrasonic sensor, an elasticity sensor, and a PH sensor. While the sensor 134 is described as one of an oil sensor, a moisture sensor, a temperature sensor, an ultrasonic sensor, an elasticity sensor, and a PH sensor, this is merely exemplary for illustrative purposes. Therefore, it is apparent to those skilled in the art that various sensors capable of measuring a skin state may be used instead.

The control unit 140 is disposed inside the casing body 111. The controller 140 is configured to control the reflective capturing unit 120 to irradiate light selected from among visible light, UV light, and IR light in the form of indirect light onto the skin, selects a light emitting part having a wavelength of IR light penetrating into the skin according to the state of the skin from the direct sensing unit 130, and controls driving of the light emitting part, for capturing.

The control unit 140 includes a control switch 141 protruding from one side surface of the casing body 111, for user manipulation, a controller 142, a reflective visible light control unit 143, a reflective UV light control unit 144, a reflective IR light control unit 145, a direct IR light control unit 146, a capturing control unit 147, and a sensor control unit 148.

The control switch 141 protrudes from the one side surface of the casing body 111. When a user presses to control light output and capturing, the control switch 141 generates a control signal.

The controller 142 is disposed inside the casing body 111. As the controller 142 is coupled to the control switch 141, the controller 142 determines whether to selectively irradiate visible light, UV light, and IR light from the reflective light emitters 122 according to a manipulation signal, and accordingly generates each indirect light emission control signal.

The indirect light emission control signal controls the reflective light emitters 122 to select one of visible light, UV light, and IR light with which to capture the color and shape of an object under measurement according to its type and shape, or irradiate the visible light, UV light, and IR light sequentially.

Further, the controller 142 is coupled to the control switch and determines whether to select one of IR wavelengths so that the direct light emitters 133 irradiate IR light in the selected wavelength directly onto the skin according to a manipulation signal, and generates a direct light emission control signal according to the wavelength.

The direct light emission control signal controls selection of an IR wavelength from among different wavelengths for different depths in the skin and irradiation of IR light in the selected wavelength through the direct light emitters 133, in order to measure a depth at which an object under measurement is located in the skin.

The controller 142 is coupled to the control switch 141 and generates a capture signal according to a manipulation signal so that the capturing module 127 determines whether to capture according to a combination of light emitted from the reflective light emitters 122 and light emitted from the direct light emitters 133, and captures an image.

Under a light irradiation condition defined by the foregoing indirect light emission control signal and direct light emission signal, an image of an object under measurement is captured according to its color, size, and shape.

Further, the controller 142 is coupled to the control switch 141 and generates a measurement control signal according to a manipulation signal, so that a sensing part of the sensor 134 selectively measures the skin.

The reflective visible light control unit 143 is disposed inside the casing body 111. The reflective visible light control unit 143 controls reflection of visible light emitted from the reflective light emitters 122 by the reflection body 123 and thus indirect irradiation of the reflected visible light according to an indirect light emission control signal generated from the controller 142 coupled to the reflective light emitters 122.

The reflective UV light control unit 144 is disposed inside the casing body 111. The reflective UV light control unit 144 controls reflection of UV light emitted from the reflective light emitters 122 by the reflection body 123 and thus indirect irradiation of the reflected UV light according to an indirect light emission control signal generated from the controller 142 coupled to the reflective light emitters 122.

The reflective IR light control unit 145 is disposed inside the casing body 111. The reflective IR light control unit 145 controls reflection of IR light emitted from the reflective light emitters 122 by the reflection body 123 and thus indirect irradiation of the reflected UV light according to an indirect light emission control signal generated from the controller 142 coupled to the reflective light emitters 122.

The indirect IR light control unit 146 is disposed inside the casing body 111. The indirect IR light control unit 146 controls irradiation of direct IR light in a selected one of a plurality of different wavelengths from the direct light emitters 133 according to a direct light emission control signal generated from the controller 142 coupled to the direct light emitters 133.

The capturing control unit 147 is disposed inside the casing body 111. The capturing control unit 147 controls capturing of the skin according to a capture signal generated from the controller 142 coupled to the capturing module 127, and transmits the captured image to the capture analysis unit 150.

The sensor control unit 148 is disposed inside the casing body 111. The sensor control unit 148 controls measurement of a skin state by a measurement sensor selected from the sensor 134 according to a measurement control signal generated from the controller 142 coupled to the sensor 134.

The capture analysis unit 150 is disposed at one side of the casing body 111. The capture analysis unit 150 is electrically coupled to the control unit 140, synthesizes images captured by the controller 140 according to a skin state, and analyzes the synthesized image through color and shape comparisons.

The capture analysis unit 150 includes a screen synthesis unit 151 for receiving images captured by the capturing module 127 through the capture control unit 147 and synthesizing the images, a color comparison unit 152, and a shape comparison unit 153.

The screen analysis unit 151 is disposed at the one side of the casing body 111. Under the condition of irradiating multi-wavelength light obtained by controlling indirect light reflected from the reflection body, which is irradiated from the reflective capturing unit 120, and direct light irradiated from the direct light sensing unit 130 in the reflective visible light control unit 143, the reflective UV light control unit 144, the reflective IR light control unit 145, and the direct IR light control unit 146, the screen synthesis unit 151 receives a plurality of images captured by the capturing module 127 coupled thereto, and synthesizes the received images.

The color comparison unit 152 is disposed at the one side of the casing body 111, compares an image synthesized by the screen synthesis unit 151 coupled to the color comparison unit 151 with basic color information generated for each color according to the type of a material generated in the skin, and diagnoses the material.

The basic color information is provided as per-color information according to the type of an object under measurement.

In the ABSCDE rule by which the risk of malignancy of melanoma is assessed, for example, it may be determined in relation to [C] whether there are various colors in the captured synthesized image by comparison.

The shape comparison unit 153 is disposed at the one side of the casing body 111, compares an image synthesized by the screen synthesis unit 151 coupled to the shape comparison unit 153 with basic shape information generated for each shape according to the type of a material generated in the skin, and diagnoses the material.

The basic shape information is provided as per-shape information according to the type of an object under measurement.

In the ABSCDE rule by which the risk of malignancy of melanoma is assessed, for example, asymmetrical shapes according to [A], irregular borders according to [B], and shapes of a diameter specified in [D] are formed as respective images and provided as the basic shape information. Therefore, an operator may compare an image with the basic shape information, to thereby facilitate material diagnosis.

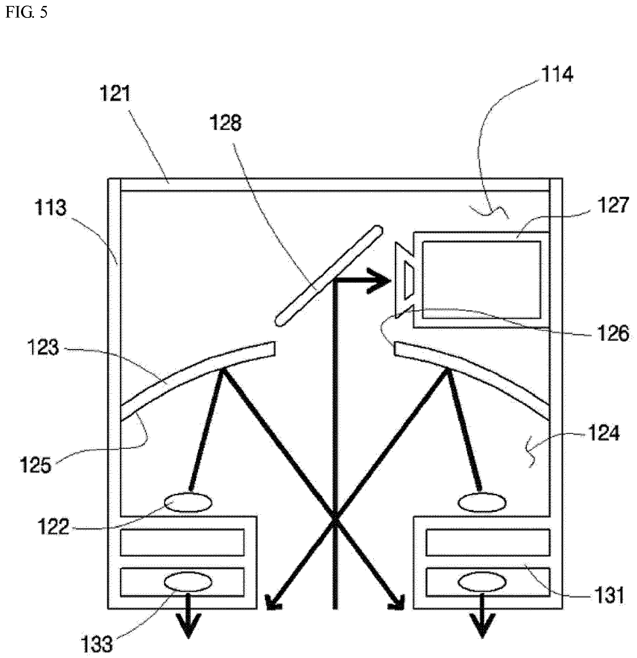

FIG. 5 is an operational state diagram illustrating the operational states of important parts in a skin state measuring apparatus using a multi-wavelength light source according to another embodiment of the present disclosure.

Referring to FIG. 5, a skin state measuring apparatus 100 using a multi-wavelength light source according to another embodiment of the present disclosure includes the casing 110, the reflective capturing unit 120, the direct light sensing unit 130, the control unit 140, and the capture analysis unit 150. The casing 110, the direct light sensing unit 130, the control unit 140, the capture analysis unit 150, and part of the reflective capturing unit 120 are identical in configuration to their counterparts of the skin state measuring apparatus 100 using a multi-wavelength light source illustrated in FIGS. 1 to 4. Therefore, only the different part of the reflective capturing unit 120 will be described below.

The reflective capturing unit 120 includes the light blocking body 121, the reflective light emitters 122, the reflection body 123, a capturing mirror 128, and the capturing module 127. The light blocking body 121, the reflective light emitters 122, and the reflection body 123 are identical in configuration to their counterparts of the skin state measuring apparatus 100 using a multi-wavelength light source illustrated in FIGS. 1 to 4. Therefore, only the different capturing mirror 128 and capturing module 127 will be described below.

The capturing mirror 128 is disposed between the light blocking body 121 and the reflection body 123. The capturing mirror 128 is positioned at a portion apart from the reflection through hole 126 by a predetermined distance and refracts an image from the center to one side direction, thereby refracting the captured image in one direction.

The capturing module 127 is disposed inside the casing body 111, and configured to capture the image refracted from the capturing mirror 128, from one side surface inside the shielding space 114 formed in the shielding body 113 toward the center. Thus, the capturing module 127 captures the skin irradiated selectively by light emitted from the reflective light emitters 122 and reflected from the reflection body 123, and direct light from the direct light emitters 133.

That is, as the capturing module 127 captures the skin through the shielding opening 115 with light refracted by the capturing mirror 128, the refraction-incurred flare phenomenon may be minimized.

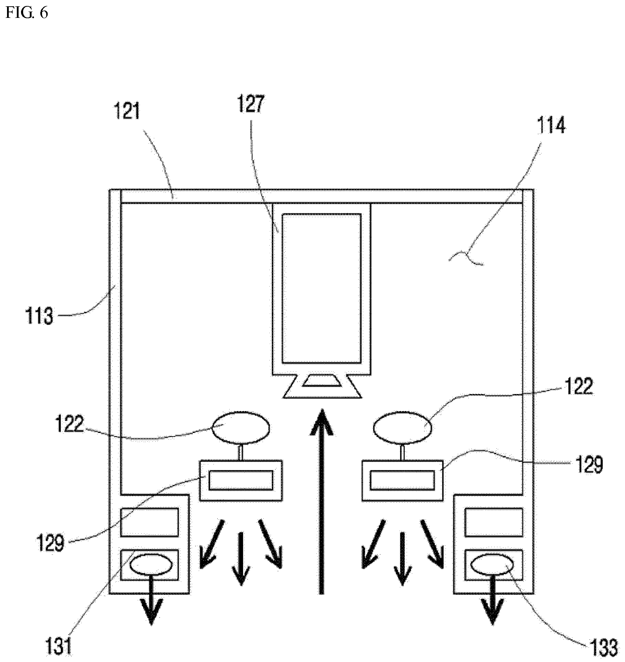

FIG. 6 is an operational state diagram illustrating the operational states of important parts in a skin state measuring apparatus using a multi-wavelength light source according to another embodiment of the present disclosure.

Referring to FIG. 6, a skin state measuring apparatus 100 using a multi-wavelength light source according to another embodiment of the present disclosure includes the casing 110, the reflective capturing unit 120, the direct light sensing unit 130, the control unit 140, and the capture analysis unit 150. The casing 110, the direct light sensing unit 130, the control unit 140, and the capture analysis unit 150 are identical in configuration to their counterparts of the skin state measuring apparatus 100 using a multi-wavelength light source illustrated in FIGS. 1 to 4. Therefore, only the different reflective capturing unit 120 will be described below.

The reflective capturing unit 120 includes the light blocking body 121, the reflective light emitters 122, diffusion plates 129, and the capturing module 127.

The light blocking body 121 is disposed inside the shielding body 113. As the light blocking body 121 is installed to close the opened other side of the shielding body 113, light is blocked from the shielding space 114 and irradiated to the outside through the shielding opening 115.

The reflective light emitters 122 are disposed apart from one side of the light blocking body 121 by a predetermined distance. In a state where the reflective light emitters 122 are provided at a plurality of positions around an outer periphery apart from a center by a predetermined distance, to irradiate visible light, UV light, and IR light toward the other side, the reflective light emitters 122 are coupled to the control unit 140, and selectively irradiate visible light, UV light, and IR light in the other direction. The reflective light emitters 122 are arranged between the light blocking body 121 and one side surface in the shielding body 113, to irradiate selected light toward the skin exposed through the shielding opening 115.

The diffusion plates 129 are arranged at one side of the reflective light emitters 122. As the diffusion plates 129 are disposed between the reflective light emitters 122 and the skin, the diffusion plates 129 diffuse light emitted from the reflective light emitters 122 in a manner that expands a light irradiation range, so that the light is irradiated as indirect light onto the skin. Since the diffusion plates 129 are fixed inside the shielding body 113 between the reflective light emitters 122 and the shielding opening 115 and converts light emitted from the reflective light emitters 122 to indirect light by diffusing and thus spreading the light, noise caused by light scattering or blur may be prevented.

The capturing module 127 is fixedly disposed at the center of a portion of the light blocking body 121. The capturing module 127 captures the skin which is irradiated selectively by light diffused through the diffusion plates 129 at the other side of the reflective light emitters 122, apart therefrom by a predetermined distance, and direct light emitted from the direct light sensing unit 130.

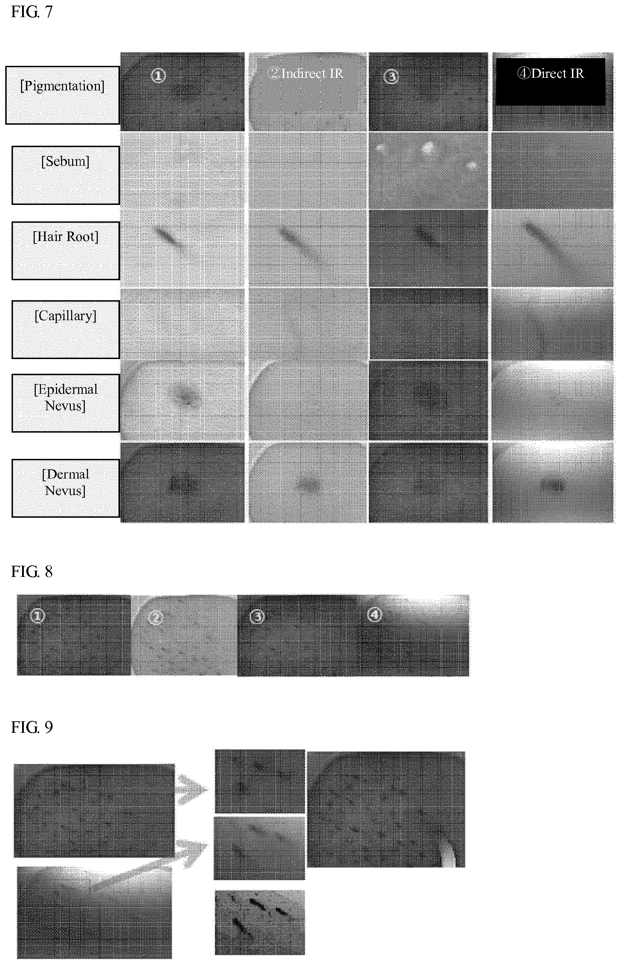

FIG. 7 illustrates pictures of images of skin states for different skin symptoms, taken in a skin state measuring apparatus using a multi-wavelength light source according to an embodiment of the present disclosure.

In FIG. 7, pictures of skin states for different skin symptoms, taken in the skin state measuring apparatus 100 using a multi-wavelength light source according to the embodiment of the present disclosure are arranged.

Among the pictures, pictures ① are images captured by irradiating reflected visible light from the reflective light emitters 122 under the control unit 140, pictures ② are images captured by irradiating reflected IR light from the reflective light emitters 122 under the control unit 140, pictures ③ are images captured by irradiating reflected UV light from the reflective light emitters 122 under the control unit 140, and pictures ④ are images captured by irradiating direct IR light from the direct light emitters 133 under the control unit 140.

As illustrated in FIG. 7, with a pigmented lesion, sebum, a hair root, a capillary, an epidermal nevus, and an intradermal nevus used as exemplary objects under measurement, the images are captured with multi-wavelength light in the skin state measuring apparatus 100 using a multi-wavelength light source.

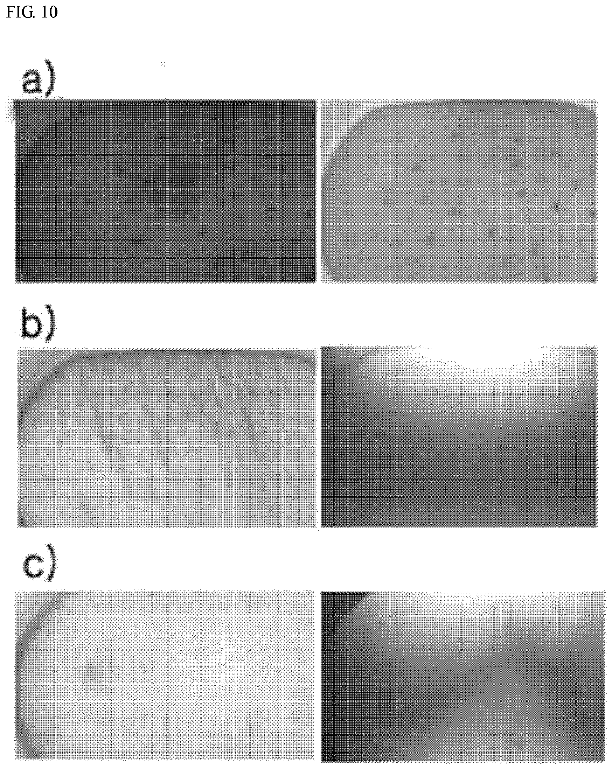

FIG. 8 illustrates pictures of a plurality of images captured for skin analysis in a skin state measuring apparatus using a multi-wavelength light source according to an embodiment of the present disclosure, FIG. 9 illustrates analysis state pictures for an image obtained by synthesizing the images of FIG. 8 in an image analysis method for skin analysis, and FIG. 10 illustrates analysis state pictures in an example of synthesizing and analyzing the images of FIG. 8 according to symptoms.

Referring to FIG. 8, ① a reflected visible light image, ② a reflected IR image, ③ a reflected UV image, and ④ a direct IR image captured by the capturing module in the skin state measuring apparatus 100 using a multi-wavelength light source according to the embodiment of the present disclosure are transmitted to the screen synthesis unit 151 of the capture analysis unit 150.

Referring to FIG. 9, to measure hair roots or a skin disorder, the epidermal state of the skin represented in ① the reflected visible light image, ② the reflected IR image, and ③ the reflected UV image, and the intradermal state of the skin represented by ④ the direct IR image are synthesized according to a skin analysis scheme.

During the synthesis, in the case of a skin disorder, a lesion is formed shallow below the surface of the skin, whereas in the case of a hair root, a hair protrudes from a hair follicle in the dermis of the skin through the epidermis of the skin. Therefore, when a skin disorder is captured, images ①, (2), and ③ are taken, with no image ④, and when a hair root is captures, all images ①, ②, ③, and ④ are taken, with a different shape in image ④.

Therefore, the root shown as overlapped shapes during synthesis of two images is displayed and distinguished from the skin disorder. In this manner, accurate information may be provided.

Referring to FIG. 10, a) corresponds to measurement of information about pigmentation by the skin state measuring apparatus 100 using a multi-wavelength light source. Since the pigmentation is not observed in ② the reflected IR image, measurement is performed by synthesizing ② the reflected IR image and ③ the reflected UV image.
b) corresponds to measurement of information about wrinkles by the skin state measuring apparatus 100 using a multi-wavelength light source. Since the wrinkles are not measured in ④ the direct IR image, measurement is performed by synthesizing ① the reflected visible light image and ④ the direct IR image.
c) corresponds to measurement of information about blood vessels by the skin state measuring apparatus 100 using a multi-wavelength light source. Since the blood vessels are measured only in ④ the direct IR image, measurement is performed by synthesizing ① the reflected visible light image and ④ the direct IR image.

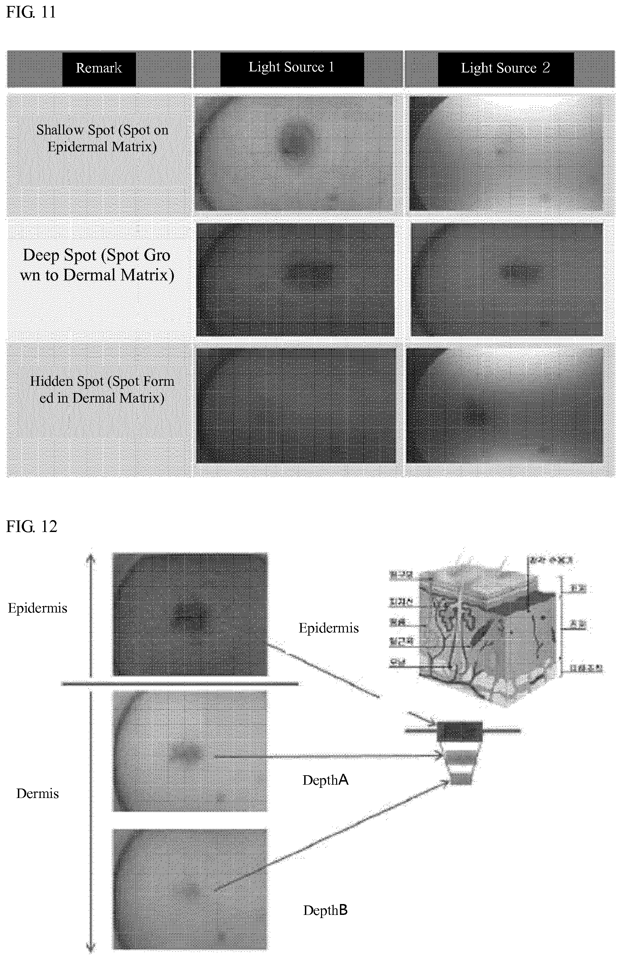

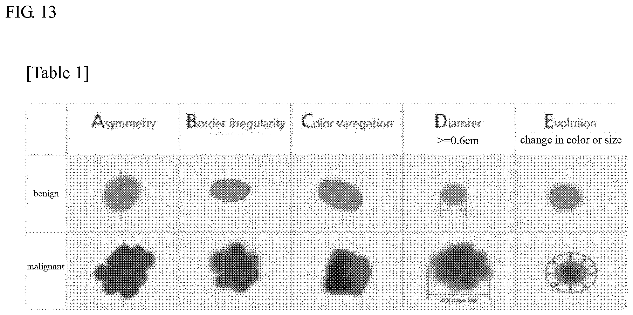

FIG. 11 illustrates pictures of images of spots generated on the skin, captured by a skin state measuring apparatus using a multi-wavelength light source according to an embodiment of the present disclosure, and FIG. 12 is a diagram illustrating a process of rendering the size and shape of a spot as a three-dimensional (3D) image by arranging the captured images of FIG. 11 according to depths into the skin and synthesizing the images.

Referring to FIG. 11, images are captured at different depths from the epidermis to the dermis of a light source 1 in wavelengths selected by selectively irradiating reflected visible light, reflected UV light, and reflected IR light through the respective reflective light emitters 122.

Further, images are captured at different depths from the epidermis to the dermis of a light source 2 in wavelengths selected by selectively irradiating light in different wavelengths through the direct light emitters 133.

A comparison between these captured images reveals that the light source 1 clearly shows the shape and color of a spot in the dermis, and the light source 2 clearly shows the shape and color of a spot deep in the dermis, which is not clear in the epidermis.

Referring to FIG. 12, images of shapes and colors at different depths from the epidermis to the dermis are selected from the per-depth images of FIG. 11, referring to the structure of the skin according to depths, and synthesized into a 3D image. Then, it is determined whether the spot is normal or melanoma and if the spot is melanoma, whether the melanoma is benign or malignant by comparing the 3D image, using the color comparison unit 152 and the shape comparison unit 153.

Further, a skin state measuring apparatus 100 using a multi-wavelength light source according to an embodiment of the present disclosure includes a light emitter for irradiating light at a measurement position of the skin, the capturing module 127, the casing body 111, the control unit 140, and the capture analysis unit 150. The capturing module 127, the casing body 111, the control unit 140, and the capture analysis unit 150 are similar to their counterparts of the skin state measuring apparatus 100 using a multi-wavelength light source illustrated in FIGS. 1 to 4. Thus, the following description is made with reference to FIGS. 1 to 4.

Further, the reflective light emitters 122 and the direct light emitters 133 illustrated in FIGS. 1 to 4 are available as the light emitter. While the light emitter is not labeled to avoid ambiguity in the description, it is apparent to those skilled in the art that any of the reflective light emitters 122 and the direct light emitters 133 is available.

The light emitter is provided with light sources of different wavelengths arranged distinguishably, so that light is selectively irradiated at a measurement position of the skin.

The capturing module 127 is provided to capture the measurement position according to the state of the light irradiated from the light emitter.

The casing body 111 is provided such that light from the light sources having different wavelengths in the light emitter is not directly irradiated but blocked.

The control unit 140 is disposed inside the casing body 111 and configured to control irradiation of the light emitter and operation of the capturing module 127. The control unit 140 is coupled to the light emitter, the capturing module 127, and the capture analysis unit 150, and controls irradiation from a light source having a selected one of different wavelengths according to the state of the skin. With the light in the selected wavelength emitted from the light emitter, the control unit 140 controls capturing of an image by the capturing module 127 and transmission of the image to the capture analysis unit 150, for image analysis.

The capture analysis unit 150 is configured to generate a synthesized image along a depth axis by synthesizing a plurality of images captured from the epidermis of the skin to a predetermined depth in the dermis of the skin, at a measurement position. The capture analysis unit 150 obtains information about the presence or absence of a spot, the size, shape, and color of the spot, and so on at the measurement position by analyzing the synthesized image, and determines the depth of the spot or whether the spot is a melanin disorder that constitutes a nevus.

While the disclosure has been shown and described with reference to certain exemplary embodiments thereof, it will be understood by those skilled in the art that various changes in form and details may be made therein without departing from the spirit and scope of the disclosure as defined by the appended claims and their equivalents.

Those skilled in the art will appreciate that the present disclosure may be carried out in other specific ways than those set forth herein without departing from the spirit and essential characteristics of the present disclosure. The above embodiments are therefore to be construed in all aspects as illustrative and not restrictive.

The scope of the disclosure should be determined by the appended claims and their legal equivalents, not by the above description, and all changes coming within the meaning and equivalency range of the appended claims are intended to be embraced therein.

## Claims

1. A skin state measuring apparatus using a multi-wavelength light source, comprising:
a casing configured to allow light of different wavelengths to be selectively irradiated in one direction onto a skin, in contact with the skin at a measurement position by shielding light emitting positions;
a reflective capturing unit disposed inside the casing, and configured to capture the skin irradiated with light, while selectively irradiating visible light, ultraviolet (UV) light, and infrared (IR) light as reflection-based indirect light onto the skin in the one direction;
a direct light sensing unit disposed inside the casing, at one side of the reflective capturing unit, and configured to sense a state of the skin, while irradiating the skin with direct light emitted from the reflective capturing unit and passed therethrough;
a control unit disposed inside the casing, and configured to control capturing and transmission of an image by controlling the reflective capturing unit and the direct light emitting unit to irradiate light selected from among the visible light, the UV light, and the IR light in the form of indirect light onto the skin and to irradiating penetrate direct light onto the skin according to the state of the skin; and
a capture analysis unit disposed at one side of the casing, coupled electrically to the control unit, and configured to synthesize images captured by the control unit according to the state of the skin and analyze the synthesized image by color and shape comparison.

2. The apparatus according to claim 1, wherein the casing unit comprises:
a casing body configured to contact the skin during skiing measurement, thereby allowing light irradiation by a user's manipulation and including, therein, a casing space opened at one side and accommodating the reflective capturing unit, the direct light sensing unit, and the control unit; and
a shielding body disposed at one side of the casing body, and including a shielding space formed at a position other than a shielding opening partially opened at a center of one side of the shielding space, with the reflective capturing unit installed in the other direction inside the shielding body, for shielding light irradiated from the reflective capturing unit, direct light emission holes at a plurality of positions at a portion of the shielding space, for allowing parts irradiating IR light of different wavelengths of the direct light sensing unit to be inserted therethrough, and a sensor installation hole for allowing a skin sensing part of the direct light sensing unit to be inserted therethroguh.

3. The apparatus according to claim 1, wherein the reflective capturing unit comprises:
a light blocking body disposed inside the casing and configured to close an opened other part of a light blocking part of the casing and emit light in the other direction;
reflective light emitters arranged, apart from one side of the light blocking body by a predetermined distance, at a plurality of positions of an outer periphery of the light emitting body apart from a center the light emitting body by a predetermined distance, to selectively irradiate the visible light, the UV light, and the IR light in the other direction, coupled to the control unit to be controlled by the control unit, and configured to selectively irradiate the visible light, the UV light, and the IR light in the other direction according to a control signal;
a reflection body disposed between the light blocking body and the reflective light emitters, and including a reflection space for accommodating the reflective light emitters therein, the reflection space being curved, surrounding the other side of the reflective light emitters and being opened at one side thereof, a reflection surface inside the reflection space, for reflecting light irradiated in the other direction by the reflective light emitters in the one direction, thereby refracting the light in the one direction, and a reflection through hole penetrating through the center of the reflection body; and
a capturing module fixed disposed at one side of the light blocking body, inserted into the reflection through hole, and configured to capture the skin in a state where light irradiated from the reflective light emitters and reflected from the reflection body and light directly emitted from the direct light sensing unit are selectively irradiated onto the skin.

4. The apparatus according to claim 1, wherein the reflective capturing unit comprises:
a light blocking body disposed inside the casing and configured to close an opened other part of a light blocking part of the casing and emit light in the other direction;
reflective light emitters arranged, apart from one side of the light blocking body by a predetermined distance, at a plurality of positions of an outer periphery of the light emitting body apart from a center the light emitting body by a predetermined distance, to selectively irradiate the visible light, the UV light, and the IR light in the other direction, coupled to the control unit to be controlled by the control unit, and configured to selectively irradiate the visible light, the UV light, and the IR light in the other direction according to a control signal;
a reflection body disposed between the light blocking body and the reflective light emitters, and including a reflection space for accommodating the reflective light emitters therein, the reflection space being curved, surrounding the other side of the reflective light emitters and being opened at one side thereof, a reflection surface inside the reflection space, for reflecting light irradiated in the other direction by the reflective light emitters in the one direction, thereby refracting the light in the one direction, and a reflection through hole penetrating through the center of the reflection body;
a capturing mirror disposed between the light blocking body and the reflection body, at one portion apart from the reflection through hole by a predetermined distance, and configured to refract a captured image from a center in one side direction; and
a capturing module disposed inside the casing, provided to capture an image refracted from the capturing mirror, at a position for capturing from one side surface toward the center, and configured to capture the skin in a state where light irradiated from the reflective light emitters and reflected from the reflection body and light directly emitted from the direct light sensing unit are selectively irradiated onto the skin.

5. The apparatus according to claim 1, wherein the reflective capturing unit comprises:
a light blocking body disposed inside the casing and configured to close an opened other part of a light blocking part of the casing and emit light in the other direction;
reflective light emitters arranged, apart from the one side of the light blocking body by a predetermined distance, at a plurality of positions of an outer periphery of the light emitting body apart from a center the light emitting body by a predetermined distance, to selectively irradiate the visible light, the UV light, and the IR light in the other direction, coupled to the control unit to be controlled by the control unit, and configured to selectively irradiate the visible light, the UV light, and the IR light in the other direction according to a control signal;
diffusion plates disposed between the reflective light emitters and the skin, at one side of the reflective light emitters, and configured to diffuse the light emitted from the reflective light emitters in an expanded light irradiation range and irradiating the light in the form of indirect light onto the skin; and
a capturing module fixed at the center of a portion of the light blocking body, apart from the other side of the reflective light emitters by a predetermined distance, and configured to capture the skin in a state where light diffused by the diffusion plates and light directly emitted from the direct light sensing unit are selectively irradiated onto the skin.

6. The apparatus according to claim 1, wherein the direct light sensing unit comprises:
a direct light blocking body disposed at the one side of the reflective capturing unit, positioned at one shielded side of the casing, on which the reflective capturing unit is installed, and including a direct light blocking through hole penetrating through the center thereof, for exposing reflected light of the reflective capturing unit and the captured skin therethrough;
a plurality of direct light emitters arranged at one side of the direct light blocking body, provided around an outer periphery of the direct light blocking through hole to emit IR light, inserted to protrude outward from a part of the casing shielding the reflective capturing unit, and configured to irradiate light directly onto the skin; and
a sensor disposed at the one side of the direct light blocking body, inserted to protrude outward from the part of the casing shielding the reflective capturing unit, and configured to measure the state of the skin in the vicinity of the skin.

7. The apparatus according to claim 1, wherein the direct light sensing unit comprises:
a direct light blocking body disposed at the one side of the reflective capturing unit, positioned at one shielded side of the casing, on which the reflective capturing unit is installed, and including a direct light blocking through hole penetrating through the center thereof, for exposing reflected light of the reflective capturing unit and the captured skin therethrough;
a plurality of direct light emitters arranged at one side of the direct light blocking body, provided around an outer periphery of the direct light blocking through hole to emit IR light in a plurality of different wavelengths, inserted to protrude outward from a part of the casing shielding the reflective capturing unit, and configured to irradiate light directly onto the skin; and
a sensor disposed at the one side of the direct light blocking body, inserted to protrude outward from the part of the casing shielding the reflective capturing unit, and configured to measure the state of the skin in the vicinity of the skin.

8. The apparatus according to claim 1, wherein the direct light sensing unit comprises:
a direct light blocking body disposed at the one side of the reflective capturing unit, positioned at one shielded side of the casing, on which the reflective capturing unit is installed, and including a direct light blocking through hole penetrating through the center thereof, for exposing reflected light of the reflective capturing unit and the captured skin therethrough;
a plurality of direct light emitters arranged at one side of the direct light blocking body, provided around an outer periphery of the direct light blocking through hole to emit IR light in a plurality of different wavelengths and visible light, inserted to protrude outward from a part of the casing shielding the reflective capturing unit, and configured to irradiate light directly onto the skin; and
a sensor disposed at the one side of the direct light blocking body, inserted to protrude outward from the part of the casing shielding the reflective capturing unit, and configured to measure the state of the skin in the vicinity of the skin.

9. The apparatus according to any of claims 6 to 8 1, wherein the sensor includes at least one of an oil sensor, a moisture sensor, an elasticity sensor a temperature sensor, an ultrasonic sensor, and a PH sensor.

10. The apparatus according to claim 1, wherein the control unit comprises:
a control switch protruding from one side surface of the casing and configured to, upon user pressing to control light output and capturing, generate a control signal;
a controller disposed inside the casing, coupled to the control switch, and configured to determine whether to selectively irradiate the visible light, the UV light, and the IR light emitted from the reflective capturing unit according to a manipulation signal and generate an indirect light emission control signal, to determine whether to directly irradiate light penetrating into the skin from the direct light sensing unit and generate a direct light emission control signal, to determine whether to capture an image according to a combination of light emitted from the reflective capturing unit and light emitted from the direct sensing unit and generate a capture signal indicating capturing, and to generate a measurement control signal to selectively measure a part of the skin, sensed by the direct light sensing unit;
a reflected visible light control unit disposed inside the casing, and configured to control the reflective capturing unit to emit reflected visible light in the form of indirect light according to the indirect light emission control signal generated from the controller coupled to the reflective capturing unit;
a reflected UV light control unit disposed inside the casing, and configured to control the reflective capturing unit to emit reflected UV light in the form of indirect light according to the indirect light emission control signal generated from the controller coupled to the reflective capturing unit;
a reflected IR light control unit disposed inside the casing, and configured to control the reflective capturing unit to emit reflected IR light in the form of indirect light according to the indirect light emission control signal generated from the controller coupled to the reflective capturing unit;
a direct IR light control unit disposed inside the casing, and configured to control the direct light sensing unit to emit direct IR light of a selected wavelength from among the plurality of different wavelengths according to the direct light emission control signal generated from the controller coupled to the direct light sensing unit;
a capturing control unit disposed inside the casing and configured to control capturing of the skin according to the capture signal generated from the controller coupled to the refractive capturing unit and transmit the captured image to the capture analysis unit; and
a sensor control unit disposed inside the casing and configured to control measurement of the state of the skin by a measurement sensor selected in the direct light sensing unit according to the measurement control signal generated from the controller coupled to the reflective capturing unit.

11. The apparatus according to claim 1, wherein the capture analysis unit comprises:
a screen synthesis unit disposed at the one side of the casing, and configured to receive a plurality of images from the control unit coupled to a capturing part of the reflective capturing unit and synthesize the received plurality of images under a condition that indirect light irradiated from the reflective capturing unit and direct light irradiated from the direct light sensing unit are controlled by the control unit and irradiated as multi-wavelength light;
a color comparison unit disposed at the one side of the casing, coupled to the screen synthesis unit, and configured to diagnose a material generated in the skin by comparing the synthesized image with basic color information generated for respective colors according to the synthesized image and a type of the material generated in the skin; and
a shape comparison unit disposed at the one side of the casing, coupled to the screen synthesis unit, and configured to diagnose the material generated in the skin by comparing the synthesized image with basic shape information generated for respective shapes according to the synthesized image and the type of the material generated in the skin.

12. A skin state measuring apparatus using a multi-wavelength light source, comprising:
a light emitter disposed with distinguishable light sources of different wavelengths to be selectively irradiated to a measurement position of a skin;
a capturing module configured to capture the measurement position, while light is irradiated from a light source;
a casing body configured to shield direct irradiation of light from the light sources of the different wavelengths to the capturing module;
a control unit disposed inside the casing body and configured to control irradiation of the light emitter and an operation of the capturing module; and
a capture analysis unit configured to synthesize a plurality of images captured at the surface of the skin to a predetermined depth into the skin at the measurement position and generate the synthesized image along a depth axis,
wherein the capture analysis unit obtains information about the presence or absence of a spot at the measurement position and the size, shape, and color of the spot by analyzing the synthesized image, and determines the depth of the spot or whether the spot is a melanin disorder forming a nevus.
